# FASCICULE DE BREVET EUROPEEN

(11) **EP 0 999 196 B1**
(45) Date de publication et mention de la délivrance du brevet: **20.10.2004**
(21) Numéro de dépôt: 99203731.7
(22) Date de dépôt: 02.11.1999
(51) Int. Cl.: C07C 17/275, C07C 17/278, C07C 17/383, C07C 19/01

(54) **Procédé de préparation d'hydrocarbures halogénés**
Verfahren zur Herstellung von halogenierten Kohlenwasserstoffen
Process for the preparation of halogenated hydrocarbons

(30) Priorité: 05.11.1998 BE 9800807
(43) Date de publication de la demande: 10.05.2000
(73) Titulaire: SOLVAY (Société Anonyme), 1050 Bruxelles (BE)
(72) Inventeur: Mathieu, Véronique, 1300 Wavre (BE); Schoebrechts, Jean-Paul, 1390 Grez-Doiceau (BE)
(74) Mandataire: Jacques, Philippe

(56) Documents cités:
- EP-A- 0 787 707
- WO-A-97/07083
- WO-A-98/50329
- WO-A-98/50330
- WO-A-99/07659
- GB-A- 2 188 929
- US-A- 3 862 978
- US-A- 5 792 893

## Description

La présente invention concerne un procédé de préparation d'hydrocarbures halogénés, comprenant au moins 3 atomes de carbone, par réaction catalytique entre un haloalcane et une oléfine, en particulier une oléfine halogénée.

L'addition d'un haloalcane sur une oléfine est une réaction bien connue, généralement dénommée réaction de télomérisation. Très souvent, des dérivés du cuivre sont utilisés pour catalyser cette réaction d'addition. Par exemple, le brevet US-A-3651019 décrit l'addition de tétrachlorure de carbone à des oléfines en présence de catalyseurs contenant du cuivre ou du fer. Ce brevet décrit des réactions en lots (procédé discontinu) et au terme de la réaction, le catalyseur n'est pas récupéré. Ceci rend le procédé peu attrayant économiquement. En cas de séparation des constituants du mélange réactionnel par distillation, le catalyseur précipiterait dans le bouilleur de la colonne de distillation, ce qui rend son recyclage difficile.

La demande de brevet WO 97/07083 décrit un procédé de préparation d'hydrocarbures halogénés par télomérisation, de préférence sous l'action catalytique du chlorure cuivreux en présence de t.butylamine comme cocatalyseur, dans lequel le mélange des produits issus de l'étape réactionnelle est séparé en plusieurs étapes. Le mélange est d'abord séparé par une distillation flash en un premier flux contenant les réactifs non convertis et le cocatalyseur et un second flux comprenant l'hydrocarbure halogéné et le catalyseur. Le catalyseur est ensuite séparé de ce second flux par filtration, puis l'hydrocarbure halogéné est purifié par distillation. Ce procédé impose une filtration du catalyseur sous forme solide, étape toujours délicate à contrôler dans une installation industrielle, en particulier dans un procédé de synthèse en continu.

La demande de brevet WO 97/05089 décrit un autre procédé de préparation d'hydrocarbures halogénés par télomérisation, sous l'action catalytique d'un catalyseur au cuivre solubilisé dans un solvant immiscible avec l'hydrocarbure halogéné recherché, ce qui permet de séparer par décantation le système catalyseur/solvant de l'hydrocarbure halogéné. La productivité de ce procédé reste toutefois très faible.

Le besoin existe dès lors de disposer d'un procédé de télomérisation qui permet de préparer des hydrocarbures halogénés avec d'excellents rendements et productivités, tout en permettant également de récupérer aisément le catalyseur et de le renvoyer au réacteur de manière simple.

La présente invention concerne dès lors un procédé de préparation d'un hydrocarbure halogéné comprenant au moins 3 atomes de carbone, comprenant les étapes suivantes :
(a) une étape de réaction entre un haloalcane et une oléfine en présence d'un catalyseur et d'un cocatalyseur dans les conditions adéquates pour produire l'hydrocarbure halogéné, le cocatalyseur étant choisi parmi les amines moins volatiles que l'hydrocarbure halogéné produit ;
(b) une étape de séparation du mélange de produits réactionnels issu de l'étape (a) choisie parmi une distillation ou une opération d'entraînement à la vapeur ou un stripping dans laquelle on obtient au moins une première fraction comprenant l'hydrocarbure halogéné et on récupère une deuxième fraction comprenant le catalyseur et le cocatalyseur ;
(c) éventuellement une étape de recyclage d'au moins une partie de la deuxième fraction à l'étape (a).
Par "fraction", on vise toute partie du mélange enrichie en certains de ses constituants, obtenue soit en flux continu ou en traitement discontinu. Par "recyclage", on vise toute opération de récupération et de réutilisation, indifféremment dans un mode continu ou discontinu.
Dans une forme spécifique de réalisation du procédé selon l'invention, on soumet, dans l'étape (b), le mélange de produits réactionnels issu de l'étape (a) à une séparation par distillation et on obtient outre la premiere fraction comprenant l'hydrocarbure halogéné et la deuxième fraction comprenant le catalyseur et le cocatalyseur, une troisième fraction comprenant des réactifs non consommés, et dans une étape (c) on recycle éventuellement à l'étape (a) également au moins une partie de la troisième fraction.

L'utilisation, comme cocatalyseur, d'une amine dont le point d'ébullition est supérieur à celui de l'hydrocarbure halogéné permet de séparer l'hydrocarbure halogéné des réactifs non consommés et du complexe actif catalyseur / cocatalyseur par distillation. Le catalyseur restant soluble dans l'amine après l'élimination des réactifs non consommés et de l'hydrocarbure halogéné, il est ainsi aisément récupéré

Dans l'étape (a) de réaction du procédé selon la présente invention, un haloalcane et une oléfine, de préférence halogénée, sont mis en réaction en présence d'un catalyseur et d'un cocatalyseur pour former un hydrocarbure halogéné, le cocatalyseur étant choisi parmi les amines moins volatiles que l'hydrocarbure halogéné produit.

Peuvent convenir comme cocatalyseur dans le procédé selon l'invention les amines comprenant de 8 à 25 atomes de carbone, en particulier les amines aliphatiques. Sont préférées les amines aliphatiques comprenant de 10 à 22 atomes de carbone. Une préférence toute particulière est accordée aux amines dont la chaîne alkyle est ramifiée et plus spécialement aux amines tert-alkyle répondant la formule générale (I) 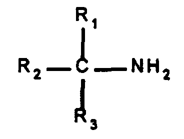 dans laquelle R₁, R₂ et R₃ représentent des groupements alkyle en C1-C8. A titre d'exemples non limitatifs d'amines présentant un point d'ébullition élevé, en particulier moins volatiles que le 1,1,1,3,3-pentachloropropane et le 1,1,1,3,3-pentachlorobutane, on peut citer notamment la nonylamine, la décylamine, la undécylamine, la dodécylamine et les collidines. Des amines répondant à la formule (I) sont notamment les amines tert-alkyles PRIMENE® 81-R et JM-T commercialisées par Rohm and Haas Company. Ces dernières sont préférées. Des mélanges d'amines peuvent également être utilisés dans le présent procédé. Les amines mises en oeuvre comme cocatalyseurs dans le procédé selon l'invention se sont en outre révélées nettement plus stables en présence du catalyseur et de l'haloalcane que les amines mises en oeuvre dans les procédés antérieurs, telles l'isopropylamine et la t.butylamine.

Les haloalcanes engagés dans le procédé selon la présente invention sont en général des composés organiques saturés. Ils possèdent de préférence de un à trois atomes de carbone et de préférence au moins 2 atomes de chlore. Ils peuvent également comprendre d'autres substituants tels que d'autres atomes d'halogène, des groupes alkyle ou halogénoalkyle. A titre d'exemples d'haloalcanes selon la présente invention, on peut citer le dichlorométhane, le chloroforme, le tétrachlorure de carbone, le 1,1,1-trichloroéthane, le 1,1,2-trichloro-1,2,2-trifluoroéthane. Le tétrachlorure de carbone est tout particulièrement préféré.

Les oléfines engagées dans le procédé selon la présente invention sont, par exemple, des oléfines linéaires ou ramifiées comprenant de 2 à 10 atomes de carbone, éventuellement substituées. Elles peuvent être substituées, par exemple, par des atomes d'halogène, des groupes alkyle, aryle ou halogénoalkyle, des groupes nitrile (CN) ou acide carboxylique (COOH). Des exemples spécifiques de telles oléfines sont, entre autres, l'éthylène, le propylène, le butylène, et le styrène et les 1-oléfines comprenant de 2 à 10 atomes de carbone.
L'oléfine est de préférence une oléfine halogénée. Les oléfines halogénées sont, de préférence, des dérivés d'un haloéthène ou d'un halopropène, eux-mêmes pouvant être substitués. Les oléfines halogénées engagées dans le procédé selon la présenté invention répondent de préférence à la formule 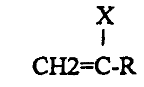 avec X=Cl ou F et R=H, Cl, F, CH₃.
Parmi ces composés, ceux où X=Cl et R=H ou CH₃ sont préférés.
De préférence, l'oléfine halogénée est une oléfine chlorée. A titre d'exemples non limitatifs d'oléfines chlorées, on peut citer le chlorure de vinyle, le chlorure de vinylidène, le trichloréthylène, les divers isomères des chloropropènes comme le 1-chloroprop-1-ène, le 2-chloroprop-1-ène et le 3-chloroprop-1-ène. Le chlorure de vinyle et le 2-chloroprop-1-ène conviennent particulièrement bien.

Les hydrocarbures halogénés obtenus selon le procédé de la présente invention appartiennent, en général, à la famille des hydrocarbures chlorés comprenant de 3 à 12 atomes de carbone. Souvent ils appartiennent à la famille des chloropropanes, des chlorobutanes ou des chloropentanes. Les atomes de carbone desdits chloropropanes, chlorobutanes et chloropentanes peuvent également être substitués par d'autres groupes fonctionnels tels que d'autres atomes d'halogène (comme des atomes de fluor, de brome ou d'iode), des groupes alkyle ou halogénoalkyle, des groupes nitrile (CN) ou acide carboxylique (COOH). De préférence, les hydrocarbures halogénés obtenus selon le procédé de la présente invention répondent à la formule générale CₙH₍₂ₙ₊₂₎₋ₚClₚ dans laquelle n est un nombre entier et possède les valeurs 3 ou 4, p est un nombre entier qui possède les valeurs 3 à 7. Des exemples de composés obtenus selon le procédé de la présente invention sont le 1,1,1,3,3-pentachloropropane, le 1,1,1,3,3-pentachlorobutane, le 1,1,1,3-tétrachloropropane, le 1,1,3,3-tétrachlorobutane, le 1,1,1,3,3,3-hexachlororopropane et le 1,1-dichloro-2-trichlorométhylpropane. Parmi ces composés, le 1,1,1,3,3-pentachloropropane, le 1,1,1,3,3-pentachlorobutane et le 1,1-dichloro-2-trichlorométhylpropane sont préférés. Le procédé selon l'invention s'applique en particulier à la fabrication du 1,1,1,3,3-pentachlorobutane au départ de tétrachlorure de carbone et de 2-chloroprop-1-ène, à la fabrication de 1,1,1,3,3-pentachloropropane au départ de tétrachlorure de carbone et de chlorure de vinyle et à la fabrication de 1,1,1,3,3,3-hexachloropropane au départ de tétrachlorure de carbone et de chlorure de vinylidène.

Le rapport molaire entre l'haloalcane et l'oléfine mis en oeuvre peut varier dans de larges mesures. Ce rapport est en général égal ou supérieur à 0,1. Avantageusement, ce rapport est égal ou supérieur à 0,5. D'une manière préférée, il est supérieur ou égal à 1. Généralement, ce rapport est inférieur ou égal à 15. Avantageusement, ce rapport est inférieur ou égal à 10. D'une manière préférée, ce rapport est inférieur ou égal à 5, voire à 3.

Le catalyseur utilisé dans la présente invention comprend de préférence au moins un composé du cuivre. Avantageusement, il s'agit d'un composé de cuivre (II). De manière particulièrement préférée, ce composé de cuivre (II) est choisi parmi le chlorure de cuivre (II), l'hydroxychlorure de cuivre (II), l'acétate de cuivre (II), l'acétylacétonate de cuivre (II) et l'hexafluoroacétylacétonate de Cu (II) et leurs mélanges. D'excellents résultats ont été obtenus avec le chlorure de Cu (II), l'acétylacétonate de cuivre (II) et l'hexafluoroacétylacétonate de Cu (II).

Le rapport molaire entre le catalyseur et l'oléfine est habituellement supérieur ou égal à 0,0001. Avantageusement, il est supérieur ou égal à 0,001. De préférence, il est supérieur ou égal à 0,005. Le rapport molaire entre le composé de Cu (II) et l'oléfine est habituellement inférieur ou égal à 1. Avantageusement, il est inférieur ou égal à 0,5. De préférence, il est inférieur ou égal à 0,1.

Le rapport molaire entre le catalyseur et le cocatalyseur est choisi de telle manière que tout le catalyseur mis en oeuvre soit dissous. Ce rapport à généralement supérieur à 0,001. Avantageusement, ce rapport molaire est supérieur à 0,01 et inférieur à 1. Une préférence est donnée pour les rapports molaires supérieurs à 0,05 et inférieurs à 0,5.

Le rapport molaire entre le cocatalyseur et l'oléfine est généralement supérieur ou égal à 0,01. De préférence, ce rapport molaire est supérieur ou égal à 0,05. Avantageusement, ce rapport molaire est supérieur ou égal à 0,1. Toutefois, ce rapport molaire est habituellement inférieur ou égal à 2. De préférence, ce rapport molaire est inférieur ou égal à 1. Avantageusement, ce rapport molaire est inférieur ou égal à 0,5.

Il est entendu que les rapports ci-dessus catalyseur / oléfine et cocatalyseur/oléfine sont exprimés, dans un procédé en discontinu, par rapport à la quantité totale d'oléfine mise en oeuvre et, dans un procédé en continu, par rapport à la quantité stationnaire d'oléfine présente dans le réacteur.

Habituellement, la réaction se déroulé à une température supérieure ou égale à la température ambiante. De préférence, la température est supérieure ou égale à 50 °C. Avantageusement, la température est supérieure ou égale à 70 °C. Toutefois, la température est généralement inférieure ou égale à 200 °C. De préférence, la température est inférieure ou égale à 175 °C. Avantageusement, la , température est inférieure ou égale à 150 °C. Une préférence toute particulière est montrée pour une température inférieure ou égale à à 120 °C, voire 100 °C.

La durée de la réaction dans un procédé en discontinu ou le temps de séjour dans un procédé en continu sont fonction de divers paramètres tels que la température de réaction, la concentration en réactifs et en catalyseur dans le mélange réactionnel et leurs rapports molaires. En général, en fonction de ces paramètres, le temps de séjour ou la durée de réaction peuvent varier de 5 minutes à 10 heures. Avantageusement, dans un procédé en discontinu, le temps de réaction est généralement supérieur ou égal à 15 minutes. Toutefois, le temps de réaction est habituellement inférieur ou égal à 10 heures avec une préférence pour les temps de réaction inférieurs ou égaux à 5 heures.

La pression est généralement choisie de façon à maintenir le milieu réactionnel en phase liquide. La pression mise en oeuvre varie en fonction de la température du milieu réactionnel. Elle est habituellement supérieure ou égale à la pression atmosphérique et inférieure ou égale à 10 bars.

La réaction de télomérisation est habituellement réalisée en phase liquide. Elle peut être réalisée en présence d'un solvant polaire tel qu'un alcool ou un nitrile. Parmi les alcools utilisables comme solvant pour la réaction, on trouve notamment le méthanol, l'éthanol, l'isopropanol et le tert-butanol. Parmi les nitriles utilisables comme solvant pour la réaction, on trouve les nitriles aliphatiques ou aromatiques. Parmi les nitriles aliphatiques, on trouve notamment l'acétonitrile, le propionitrile ou l'adiponitrile. Parmi les nitriles aromatiques, on trouve notamment le benzonitrile ou le tolunitrile. Le propionitrile et l'adiponitrile sont préférés. La quantité de solvant engagé dans la réaction n'est pas critique. Une solution trop diluée n'est cependant pas favorable à un rendement et à un taux de conversion élevés. De préférence, le rapport molaire du solvant à l'oléfine est égal ou supérieur à 0,05. Avantageusement, ce rapport est égal ou supérieur à 0,1. Le rapport molaire du solvant à l'oléfine est en général égal ou inférieur à 30. Avantageusement, il est égal ou inférieur à 20. D'une manière préférée, ce rapport est égal ou supérieur à 1 et égal ou inférieur à 15. Toutefois, dans le procédé selon l'invention, la présence d'un cocatalyseur en quantité suffisante pour dissoudre le catalyseur permet généralement de réaliser la réaction en absence de solvant.

Dans le procédé selon l'invention on effectue une étape (b) de séparation. La technique de séparation est choisie parmi une distillation ou une opération d'entraînement à la vapeur ou un stripping. Parmi ces techniques, une distillation est préférée.

Le cas échéant, la distillation est réalisée de manière connue en soi, dans une ou, de préférence, plusieurs colonnes de distillation. De préférence, elle est menée sous pression réduite.

De manière préférée, le procédé selon l'invention comprend également une étape (c), dans laquelle on renvoie à l'étape (a) tout ou partie de la fraction contenant le catalyseur et le cocatalyseur, éventuellement après purification supplémentaire. D'autres fractions obtenues lors de l'étape (b) peuvent également être renvoyées à l'étape (a), notamment, le cas échéant, une fraction contenant les réactifs non consommés, éventuellement après purification supplémentaire.

En cas de diminution du taux de conversion de l'oléfine lorsqu'on effectue une étape de recyclage (c), l'ajout d'un appoint de cocatalyseur frais et éventuellement de catalyseur frais permet de retrouver le taux de conversion initial. Ceci permet de limiter de manière très importante la consommation de catalyseur et de cocatalyseur, ainsi que la quantité de déchets à éliminer.

Un des principaux avantages du procédé selon l'invention est que le catalyseur reste à l'état dissous tout au long de l'étape (b) de séparation, ce qui évite une manipulation compliquée de composés solides. Un autre avantage du procédé est qu'il permet d'obtenir les hydrocarbures chlorés désirés avec une productivité et un rendement très élevés.

Les hydrocarbures halogénés obtenus selon le procédé de l'invention sont des précurseurs des analogues fluorés correspondants, lesquels peuvent être facilement obtenus par traitement avec du fluorure d'hydrogène en présence d'un catalyseur tel qu'un sel d'antimoine, un sel de titane, un sel de tantale, un sel d'étain ou un dérivé du chrome.

L'exemple ci-après illustre l'invention de manière non limitative.

### Exemple

On a préparé du 1,1,1,3,3-pentachlorobutane au départ de 2-chloroprop-1-ène (2-CPe) et de tétrachlorure de carbone, par réaction entre ces réactifs en présence d'acétylacétonate de cuivre (II) et d'amine Primène® JM-T. Pour ce faire, on a introduit le 2-CPe, le CCl₄, le catalyseur et l'amine dans un rapport molaire 1 2.2 / 0.02 / 0.22 dans un autoclave de 300 ml dont les parois internes sont recouvertes de téflon. L'appareil a ensuite été fermé hermétiquement, placé dans un four vertical et la température a été augmentée progressivement et maintenue à 90 °C pendant 1.5 heure. L'agitation a été assurée par un barreau magnétique placé dans le fond de l'autoclave. En fin de réaction, on a laissé refroidir l'autoclave, un échantillon de liquide a été prélevé à la seringue et dosé par une méthode chromatographique. La conversion du 2-CPe était de 99%.

On a alors soumis le mélange des produits réactionnels à une distillation. Le CCl₄ excédentaire a d'abord été soutiré par distillation, puis le 1,1,1,3,3-pentachlorobutane a ensuite été soutiré par distillation sous pression réduite. La solution restant dans le bouilleur de la colonne, essentiellement constituée du catalyseur dissous dans le cocatalyseur, a été transférée dans le réacteur de télomérisation, ainsi que le CCl₄ distillé. On y a rajouté du 2-chloroprop-1-ène et du tétrachlorure de carbone en quantité telle que le rapport molaire entre le 2-CPe, le CCl₄, le catalyseur et l'amine était à nouveau de 1/1,2/0,02/0,22, puis on a reproduit l'étape réactionnelle. Après 1,5 heure de réaction à 90 °C, le taux de conversion du 2-chloroprop-1-ène était de 92 %.

## Revendications

1. Procédé de préparation d'un hydrocarbure halogéné comprenant au moins 3 atomes de carbone, comprenant les étapes suivantes :
(a) une étape de réaction entre un haloalcane et une oléfine en présence d'un catalyseur et d'un cocatalyseur dans les conditions adéquates pour produire l'hydrocarbure halogéné, le cocatalyseur étant choisi parmi les amines moins volatiles que l'hydrocarbure halogéné produit ;
(b) une étape de séparation du mélange de produits réactionnels issus de l'étape (a) choisie parmi une distillation, une opération d'entraînement à la vapeur ou un stripping dans laquelle on obtient au moins une première fraction comprenant l'hydrocarbure halogéné et on récupère une deuxième fraction comprenant le catalyseur et le cocatalyseur ;
(c) éventuellement une étape de recyclage d'au moins une partie de la deuxième fraction à l'étape (a).

2. Procédé selon la revendication 1 lequel le cocatalyseur est une amine aliphatique comprenant de 8 à 25 atomes de carbone.

3. Procédé selon la revendication 1 ou 2 dans lequel le cocatalyseur est choisi parmi la nonylamine, la décylamine, la undécylamine, la dodécylamine.

4. Procédé selon la revendication 2 dans lequel le cocatalyseur est une amine tert-alkyle répondant la formule générale (I) 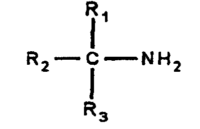 dans laquelle R₁, R₂ et R₃ représentent des groupements alkyle en C1-C8

5. Procédé selon l'une quelconque des revendications 1 à 4 dans lequel le catalyseur comprend au moins un composé du cuivre.

6. Procédé selon l'une quelconque des revendications 1 à 4 dans lequel l'étape (b) est une étape de distillation.

7. Procédé selon l'une quelconque des revendications 1 à 6 dans lequel on obtient à l'étape (b) en outre une troisième fraction comprenant des réactifs non consommés et dans lequel on renvoie à l'étape (a) au moins une partie de la deuxième et /ou de la troisième fraction.

8. Procédé selon l'une quelconque des revendications 1 à 7, dans lequel l'oléfine est une oléfine halogénée.

9. Procédé selon l'une quelconque des revendications 1 à 8, appliqué à la fabrication du 1,1,1,3,3-pentachloropropane ou du 1,1,1,3,3-pentachlorobutane.

10. Procédé selon l'une quelconque des revendications 1 à 9, comprenant en outre une étape de traitement de l'hydrocarbure halogéné obtenu avec du fluorure d'hydrogène en présence d'un catalyseur.

## Patentansprüche

1. Verfahren zur Herstellung eines wenigstens 3 Kohlenstoffatome umfassenden halogenierten Kohlenwasserstoffes, das die folgenden Stufen umfaßt:
(a) eine Umsetzungsstufe zwischen einem Halogenalkan und einem Olefin in Gegenwart eines Katalysators und eines Cokatalysators unter zur Ausbildung des halogenierten Kohlenwasserstoffes geeigneten Bedingungen, wobei der Cokatalysator unter den Aminen ausgewählt wird, die weniger flüchtig sind als der gebildete halogenierte Kohlenwasserstoff;
(b) eine Stufe zur Auftrennung des aus der Stufe (a) erhaltenen Gemisches von Reaktionsprodukten, ausgewählt unter einer Destillation, einer Wasserdampf-Mitnahmebehandlung oder einem Strippen, worin wenigstens eine erste Fraktion erhalten wird, die den halogenierten Kohlenwasserstoff umfaßt, und eine zweite Fraktion gewonnen wird, die den Katalysator und den Cokatalysator umfaßt;
(c) gegebenenfalls eine Stufe der Rezyklierung wenigstens eines Teiles der zweiten Fraktion zur Stufe (a).

2. Verfahren nach Anspruch 1, worin der Cokatalysator ein 8 bis 25 Kohlenstoffatome umfassendes aliphatisches Amin ist.

3. Verfahren nach Anspruch 1 oder 2, worin der Cokatalysator unter Nonylamin, Decylamin, Undecylamin oder Dodecylamin ausgewählt wird.

4. Verfahren nach Anspruch 2, worin der Cokatalysator ein tertiäres Alkylamin entsprechend der allgemeinen Formel (I) 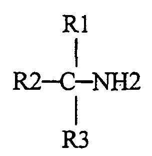 ist, worin R₁, R₂ und R₃ C₁-C₈-Alkylgruppen darstellen.

5. Verfahren nach einem der Ansprüche 1 bis 4, worin der Katalysator wenigstens eine Kupferverbindung umfaßt.

6. Verfahren nach einem der Ansprüche, 1 bis 4, worin die Stufe (b) eine Destillationsstufe ist.

7. Verfahren nach einem der Ansprüche 1 bis 6, worin in der Stufe (b) zusätzlich eine dritte Fraktion erhalten wird, die die nicht verbrauchten Reaktionskomponenten umfaßt und worin wenigstens ein Teil der zweiten und/oder der dritten Fraktion zur Stufe (a) zurückgeführt wird.

8. Verfahren nach einem der Ansprüche 1 bis 7, worin das Olefin ein halogeniertes Olefin ist.

9. Verfahren nach einem der Ansprüche 1 bis 8, angewandt auf die Herstellung von 1,1,1,3,3-Pentachlorpropan oder von 1,1,1,3,3-Pentachlorbutan.

10. Verfahren nach einem der Ansprüche 1 bis 9, das zusätzlich eine Stufe der Behandlung des erhaltenen halogenierten Kohlenwasserstoffes mit Fluorwasserstoff in Gegenwart eines Katalysators umfaßt.

## Claims

1. Process for preparing a halogenated hydrocarbon, comprising at least 3 carbon atoms, comprising the following steps:
(a) a reaction step between a haloalkane and an olefin in the presence of a catalyst and a co-catalyst under conditions which are suitable for producing the halogenated hydrocarbon, the co-catalyst being chosen from amines which are less volatile than the halogenated hydrocarbon;
(b) a separation step of the mixture of reaction products from step selected from a distillation, a vapour stripping or a stripping (a) wherein at least a first fraction comprising the halogenated hydrocarbon is obtained and a second fraction comprising the catalyst and the co-catalyst is recovered;
(c) optionally a recycling step of a least a part of the second fraction into step (a).

2. Process according to Claim 1, in which the co-catalyst is an aliphatic amine comprising from 8 to 25 carbon atoms.

3. Process according to Claim 1 or 2, in which the co-catalyst is chosen from nonylamine, decylamine, undecylamine and dodecylamine.

4. Process according to Claim 2, in which the co-catalyst is a tert-alkyl amine corresponding to the general formula (I) 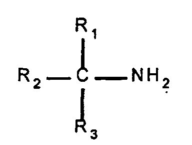 in which R₁, R₂ and R₃ represent C1-C8 alkyl groups.

5. Process according to any one of Claims 1 to 4, in which the catalyst comprises at least one copper compound.

6. Process according to any one of Claims 1 to 4 , in which step (b) is distillation step.

7. Process according to any one of Claims 1 to 6, in which in step (b) in addition, a third fraction comprising not consumed reagents is obtained and at least a part of the second fraction and/or of the third fraction is returned to step (a).

8. Process according to any one of Claims 1 to 7, in which the olefin is a halogenated olefin.

9. Process according to any one of Claims 1 to 8, applied to the manufacture of 1,1,1,3,3-pentachloropropane or 1,1,1,3,3-pntachlorobutane.

10. Process according to any one of Claims 1 to 9 comprising in addition a treatment step of the obtained halogenated hydrocarbon with hydrogen fluoride in the presence of a catalyst.
